(19) Europäisches Patentamt
European Patent Office
Office européen des brevets

(11) EP 3 281 673 A1

(12) EUROPEAN PATENT APPLICATION
published in accordance with Art. 153(4) EPC

(43) Date of publication:
14.02.2018 Bulletin 2018/07

(51) Int Cl.:
A61N 5/10 (2006.01)

(21) Application number: 15888490.8

(22) Date of filing: 09.04.2015

(86) International application number:
PCT/JP2015/061085

(87) International publication number:
WO 2016/162998 (13.10.2016 Gazette 2016/41)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(71) Applicant: Mitsubishi Electric Corporation
Chiyoda-ku
Tokyo 100-8310 (JP)

(72) Inventors:
• IWATA, Takaaki
Tokyo 100-8310 (JP)
• HARADA, Hisashi
Tokyo 100-8310 (JP)

(74) Representative: Sajda, Wolf E.
Meissner Bolte Patentanwälte
Rechtsanwälte Partnerschaft mbB
Postfach 86 06 24
81633 München (DE)

(54) THERAPY PLANNING APPARATUS AND PARTICLE RADIATION THERAPY APPARATUS

(57) A treatment planning apparatus includes an overall data management unit for storing a target irradiation dose distribution to be formed in an irradiation object, a broad irradiation parameter calculation unit and a scanning irradiation parameter calculation unit for cooperatively calculating and determining operational parameters for devices, such as an accelerator and an irradiation nozzle, to operate during a broad irradiation and an scanning irradiation, respectively, so that the sum of irradiation doses imparted by both broad irradiation and scanning irradiation forms the target irradiation dose distribution.

FIG. 1

EP 3 281 673 A1

**Description**

Technical Field

The present invention relates to a particle beam therapy system that performs particle beam irradiation for cancer treatment and the like as application of a particle beam, and more particularly to a treatment planning apparatus for the therapy system.

Background Art

Particle beam irradiation methods for particle beam therapy systems are roughly categorized into two methods: a broad irradiation method and a scanning irradiation method. In a wobbler method, which is one type of a broad irradiation method, a charged particle beam is spread by being scanned with scanning electromagnets in a circular pattern and is shaped to irradiate an irradiation object in accordance with the shape thereof.

The scanning irradiation method is for performing irradiation by scanning a charged particle beam across an irradiation object with scanning electromagnets. In a scanning irradiation method, irradiation is generally performed with the irradiation dose being controlled for each of irradiation points.

In a broad irradiation method typified by the wobbler method, on the other hand, irradiation is performed with the irradiation dose being not controlled for each of irradiation points but controlled for an irradiation region as a whole.

The wobbler method is a conventionally used irradiation method, and has a merit in that there have been many actual results in clinical practice but has a demerit in that a bolus (typically formed of resin) needs to be fabricated to mimic a distal shape of a diseased site on a patient-by-patient basis.

The scanning irradiation method, although having a merit of performing a three-dimensional irradiation with increased flexibility, has some demerits such as in that there have yet been fewer actual results in clinical practice than the broad irradiation method because the scanning irradiation method is a recent technology and in that optimization calculation takes time to formulate a treatment plan.

From a viewpoint of development of irradiation apparatuses in particle beam therapy systems, an irradiation nozzle for the wobbler method was first developed and then an irradiation nozzle for the scanning irradiation method was developed. Buyers of those days were requested to alternatively decide, as an irradiation apparatus in one treatment room, whether an irradiation nozzle for the broad irradiation method or an irradiation nozzle for the scanning irradiation method.

After that, there were proposed an irradiation nozzle that achieved both broad irradiation and scanning irradiation with one irradiation nozzle, and there were also proposed a gantry that was provided with two irradiation lines (Patent Document 1), thus increasing flexibility in the treatment methods.

In an irradiation nozzle provided to one irradiation line, there is also known an irradiation nozzle that operates in either one of a broad irradiation configuration and a scanning irradiation configuration and retracts the other unused configuration not to interrupt the charged particle beam irradiation (Patent Documents **2** and **3**).

Prior Art Document

Patent Documents

    Patent Document **1**: JP 2010-158479 A;
    Patent Document **2**: JP 2009-236867 A;
    Patent Document **3**: WO2013/011583 A1

Summary of the Invention

Problem that the Invention is to Solve

While a gantry equipped with two irradiation lines and an irradiation nozzle capable of performing the broad irradiation and the scanning irradiation with one irradiation nozzle have been thus put into practical use, usage thereof is no more than that one of the irradiation methods is alternatively determined for one patient. This poses a problem in that the demerit of the broad irradiation method still remains when the broad irradiation method is selected or the demerit of the scanning irradiation method still remains when the scanning irradiation method is selected.

In consideration of the above problem, the present invention is aimed at achieving a highly accurate and highly flexible irradiation that utilizes the respective merits of the broad irradiation and the scanning irradiation by performing these irradiations for one diseased site.

Means for Solving the Problem

The present invention offers a treatment planning apparatus configured to formulate a treatment plan that allows a particle beam therapy system to irradiate an irradiation object with a particle beam extracted from an accelerator, by switching between a scanning irradiation from a scanning irradiation nozzle mounted with scanning irradiation use parts for the scanning irradiation that is performed while shifting the particle beam and controlling an irradiation dose imparted to each of points in the irradiation object and a broad irradiation from a broad irradiation nozzle mounted with broad irradiation use parts for the broad irradiation that is performed by controlling a total irradiation dose imparted to a region in the irradiation object. The treatment planning apparatus includes
an overall data management unit configured to store a target irradiation dose distribution to be formed in the irradiation object;
broad irradiation parameter calculation unit configured to calculate operational parameters for respective devices, such as the accelerator and the broad irradiation nozzle, to operate during the broad irradiation; and
a scanning irradiation parameter calculation unit configured to calculate operational parameters for the respective devices, such as the accelerator and the scanning irradiation nozzle, to operate during the scanning irradiation, wherein the broad irradiation parameter calculation unit and the scanning irradiation parameter calculation unit is adapted to cooperatively calculate and determine the operational parameters for the respective devices, such as the accelerator and the broad irradiation nozzle, to operate during the broad irradiation and the operational parameters for the respective devices, such as the accelerator and the scanning irradiation nozzle, to operate during the scanning irradiation, so that the sum of irradiation doses imparted by both broad irradiation and scanning irradiation forms the target irradiation dose distribution.

Advantage of the Invention

A treatment planning apparatus according to the present invention enables a treatment plan to be formulated in a short time and allows for constructing a particle beam therapy system that imparts irradiation doses with high accuracy to diseased sites of various shapes.

Brief Description of the Drawings

| FIG. 1 | is a block diagram showing an overall configuration of a particle beam therapy system including a treatment planning apparatus according to Embodiment 1 of the present invention; |
| FIG. 2 | is a side view showing a scanning irradiation use parts mounting configuration of an example of an irradiation nozzle equipped in the particle beam therapy treatment system to which the treatment planning apparatus of the present invention is applied; |
| FIG. 3 | is a side view showing a broad irradiation use parts mounting configuration of the example of the irradiation nozzle equipped in the particle beam therapy system to which the treatment planning apparatus of the present invention is applied; |
| FIG. 4 | is a schematic diagram showing an example of an irradiation apparatus equipped with two irradiation lines that is installed in the particle beam therapy system to which the treatment planning apparatus of the present invention is applied; |
| FIGS. 5A and 5B | show schematic illustrations of an irradiation based on a treatment plan formulated by the treatment planning apparatus according to Embodiment 1 of the present invention; |
| FIG. 6 | is a flow diagram showing an operation flow of the treatment planning apparatus according to Embodiment 1 of the present invention; |
| FIG. 7 | shows a schematic illustration of an irradiation based on a treatment plan formulated by the treatment planning apparatus according to Embodiment 2 of the present invention; and |
| FIG. 8 | shows a schematic illustration of an irradiation based on a treatment plan formulated by the treatment planning apparatus according to Embodiment 3 of the present invention. |

Embodiments for Carrying Out the Invention

Embodiment 1

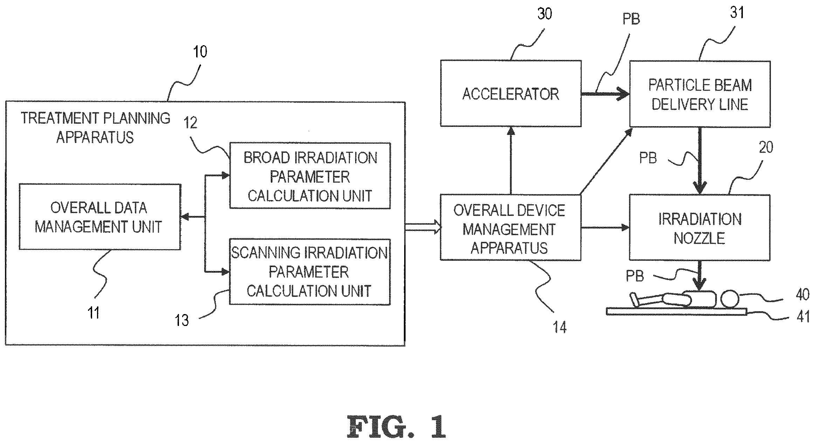

FIG. 1 is a block diagram showing an overall configuration of a particle beam therapy system including a treatment planning apparatus according to Embodiment 1 of the present invention. A particle beam *PB* extracted from an accelerator 30 is guided to an irradiation nozzle 20 through a particle beam delivery line 31.

The irradiation nozzle 20 is provided with various parts and configured to switch between a broad irradiation and a scanning irradiation to irradiate with the particle beam *PB* a diseased site of a patient 40, an irradiation object. Meanwhile, a treatment plan how to irradiate the diseased site with the particle beam is in advance formulated appropriately to the diseased site of the patient by a treatment planning apparatus 10.

The treatment planning apparatus 10 determines and stored therein operational parameters for respective devices, such as the accelerator 30, the particle beam delivery lines 31, and the irradiation nozzle 20, of the particle beam therapy system for it to perform irradiation in accordance with the formulated treatment plan.

The operational parameters are sent from, for example, an overall device management apparatus 14 to the respective devices for them to be controlled so as to operate in accordance with the treatment plan during treatment , i.e., during irradiating the patient diseased site with the particle beam.

FIG. 1 also shows a schematic configuration of the treatment planning apparatus 10 according to the present invention. An overall data management unit 11 stores therein irradiation distribution data for the patient diseased site to be irradiated. The data contains, for example, a three-dimensional irradiation region and an irradiation dose distribution in the irradiation region.

The irradiation dose distribution is referred here to as a target irradiation dose distribution. The treatment planning apparatus 10 is further has a broad irradiation parameter calculation unit 12 and a scanning irradiation parameter calculation unit 13.

The broad irradiation parameter calculation unit 12 calculates parameters for the respective devices to perform irradiation using a broad irradiation method (hereinafter, referred to as broad irradiation), and the scanning irradiation parameter calculation unit 13 calculates parameters for the respective devices to perform irradiation using a scanning irradiation method (hereinafter, referred to as scanning irradiation).

The treatment planning apparatus of the present invention formulates the treatment plan so that the target irradiation dose distribution is formed by combination of the broad irradiation and the scanning irradiation, as will be described later. #

The operational parameters for the respective devices thus calculated according to the treatment plan is stored, for example, in the overall data management unit 11, and the operational parameters stored are sent from, for example, an overall device management apparatus 14 to the respective devices for them to operate in accordance with the parameters during treatment.

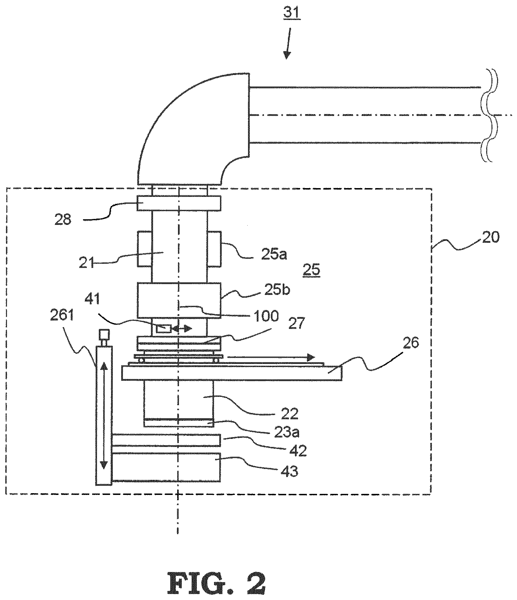

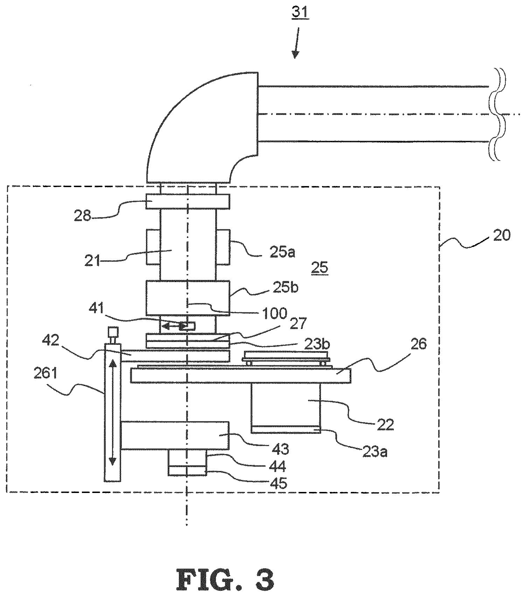

FIGS. 2 and 3 show examples of configurations of the irradiation nozzle capable of the scanning irradiation and the broad irradiation by switching therebetween. FIG. 2 shows an example of a scanning irradiation configuration of the irradiation nozzle. This configuration is disclosed, for example, in Patent Document 3.

As shown in FIG. 2, the particle beam extracted from the accelerator 30 is delivered to the irradiation nozzle 20 through the particle beam delivery line 31 configured with vacuum ducts and deflectors; then passes through a connecting vacuum duct 21 and a scanning irradiation use vacuum duct 22 provided in the irradiation nozzle 20 for keeping the vacuum condition; and is extracted to the atmosphere from a beam extraction window 23a to irradiate the diseased site, the irradiation object. The particle beam is scanned across the irradiation object by scanning electromagnets 25a and 25b (also referred to as scanning electromagnets 25 collectively).

The irradiation nozzle 20 is further provided with a vacuum duct moving mechanism 26 for retracting the scanning irradiation use vacuum duct 22 from the beam line 100, which is the beam center line indicated by the dot-dash line, to switch the parts arrangement to that for the broad irradiation; a connection flange plane 27 of the vacuum duct 21; a gate valve 28 for separating the vacuum condition in the vacuum duct 21 at a position immediately upstream the scanning electromagnets 25; and a scatterer 41 for scattering the particle beam in conformity to the irradiation region.

The irradiation nozzle is further provided with a ridge filter 42 for spreading out the Bragg peak of the particle beam in the depth-wise direction and a range shifter 43 for adjusting the penetration range of the particle beam. The ridge filter 42 and the range shifter 43 here are attached to a ridge filter moving mechanism 261 so as to be movable in the direction parallel to the beam line 100.

Next, the operation of the irradiation nozzle will be described. In a case of the scanning irradiation, in order to make small the beam spot size at beam irradiation points by suppressing scattering of the particle beam as far as possible, a vacuum duct is generally disposed close up to the beam irradiation points.

Since the scatterer 41 is unnecessary in this case, it is retracted to the side of the beam line 100 within the vacuum duct 21. In a case of the particle beam being a proton beam, the ridge filter 42 is unnecessary for the scanning irradiation; however, a ridge filter may be used in some cases for the other particle beam, to increase the energy width slightly.

For example, in a case of a heavy particle beam such as a carbon ion beam, since the beam has a very sharp Bragg peak width compared to a proton beam, a ridge filter may be used to form a spread-out Bragg peak (SOBP) in order to irradiate a certain depth width (several mm) in one scan thereby to reduce irradiation time.

Note that the ridge filter is for spreading out the Bragg peak width to several mm and its bar ridge height may be shorter than the SOBP width even though the ridge filter is disposed at a position not away from the irradiation object. Accordingly, a ridge filter can be used for the heavy particle beam that is manufactured much easier than that for the broad irradiation.

Furthermore, the penetration depth (penetration range) of the particle beam depends on the energy of the particle beam; hence, varying the energy of the particle beam is necessary to vary the penetration range thereof. Performing change of the energy only by energy adjustment of the accelerator poses a problem of taking time to change the energy. For that reason, a range shifter for reducing the energy of the particle beam is used in some cases to vary the energy of the particle beam.

Considering the fact that the particle beam is scattered by the range shifter, the range shifter is desirably disposed as downstream as possible, in other words, in a position as close as possible to the irradiation object. Accordingly, in performing the scanning irradiation using the ridge filter **42** and the range shifter **43,** they are preferably arranged as shown in **FIG. 2.**

Switching from the scanning irradiation to the broad irradiation is described next. **FIG. 3** shows a broad irradiation configuration of the irradiation nozzle **20** switched from the scanning irradiation configuration shown in **FIG. 2.** In the scanning irradiation, the scanning irradiation use vacuum duct **22** is disposed at a position close up to the irradiation object to prevent the beam spot size from increasing.

In the broad irradiation, on the other hand, the ridge filter **42** needs to be disposed at a position away from the irradiation object because the particle beam necessarily has a largely increased energy width. In the particle beam therapy system according to Embodiment **1,** all of the scanning irradiation use vacuum duct **22** and its accompanying parts disposed downstream the scanning electromagnets **25** are dismounted and retracted away from the beam line **100** to ensure a long space.

In **FIG.2,** the scanning irradiation use vacuum duct **22** is detachable from the vacuum duct **21** at the flange plane **27** disposed downstream the scanning electromagnets **25.** Moreover, when the scanning irradiation use vacuum duct **22** is dismounted from the connection flange, the scanning irradiation use vacuum duct **22** is slid and retracted away from the beam line **100** to a position not to easily overlap with the beam line **100,** by the vacuum duct moving mechanism **26** provided with a driving base and a driving rail for supporting the scanning irradiation use vacuum duct **22.**

After the scanning irradiation use vacuum duct **22** is dismounted, since the connection flange plane **27** becomes an end plane for the vacuum condition, a beam extraction window **23b** is attached to the flange plane **27** as shown in **FIG. 3.**

The ridge filter **42** is lifted up toward the flange plane **27** and placed at a position just beneath a beam extraction window **23b** with the ridge filter moving mechanism **261** through the space created by sliding the scanning irradiation use vacuum duct **22** with the vacuum duct moving mechanism **26.**

At this time, the ridge filter **42** is exchanged from that for scanning irradiation use to that for broad irradiation use. Moreover, the range shifter **43** is moved up and down, if needed, for a bolus **44** and a patient collimator **45** to be mounted.

Furthermore, the scatterer **41,** which is retracted from the beam line **100** in the case of scanning irradiation, is moved to the beam line **100.** In a broad irradiation using the wobbler method, the particle beam is not necessarily spread by the scatterer **41** but is spread by being scanned by the scanning electromagnets **25** in a circular pattern to perform the irradiation.

In this way, the broad irradiation is enabled. The bolus **44** and the patient collimator **45** can be easily mounted by attaching insertion holders therefor to the bottom face of the range shifter **43** with rails or the like. The ridge filter **42** and the range shifter **43** can be inserted using a linear translation mechanism or a rotational translation mechanism driven by air or a motor.

While the scanning irradiation use vacuum duct **22** is slidably retracted in the above configuration, providing a rotatable support mechanism also allows the retraction and the insertion of the vacuum duct **22** to be switched to each other by rotating the support mechanism.

If no vacuum separation plane was provided upstream the scanning irradiation use vacuum duct **22** and the scanning irradiation use vacuum duct **22** communicated to the upstream, retraction of the scanning irradiation use vacuum duct **22** would result in breakage of the vacuum condition throughout the beam lines. In this case, it takes time to increase the degree of vacuum.

Hence, the gate valve **28** is preferably disposed immediately upstream the scanning electromagnets **25.** The gate valve may also be disposed in a position immediately downstream the scanning electromagnets **25.** When the scanning irradiation use vacuum duct **22** is dismounted, closing the gate valve **28** allows influence to the degree of vacuum to be limited to only the downstream of the gate valve **28**.

At that time, configuring the gate valve **28** to have also a function of a final beam extraction window eliminates the need to attach the beam extraction window **23b** anew, thereby reducing time for the switching between the broad irradiation and the scanning irradiation.

As described above, the irradiation nozzle **20** thus allows the particle beam irradiation to be switched between the scanning irradiation and the broad irradiation, as shown in **FIGS. 2** and **3**.

An irradiation apparatus capable of irradiation by switching between the broad irradiation and the scanning irradiation may be configured to have two irradiation lines: a first irradiation line having an irradiation nozzle for the broad irradiation and a second irradiation line having an irradiation nozzle for the scanning irradiation. Such an irradiation apparatus has already been disclosed in Patent Document **1.**

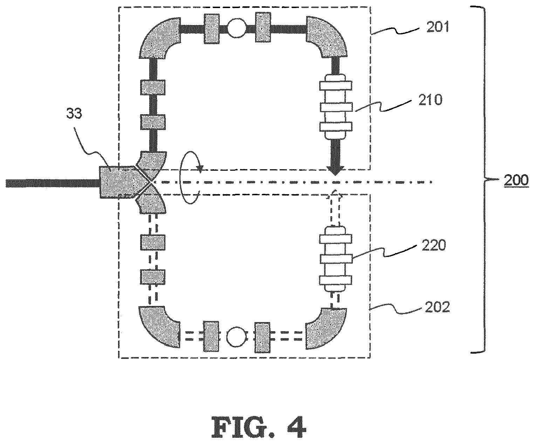

**FIG. 4** is a schematic diagram showing an example of an irradiation apparatus having two irradiation lines. The irradiation apparatus, which is designated at **200**, is a so-called gantry type irradiation apparatus and is provided in the gantry with two irradiation lines: a first irradiation line **201** and a second irradiation line **202**. Delivery of the particle beam is switched between the first irradiation line **201** and the second irradiation line **202** by a delivery line switching device **33**.

When delivery of the particle beam is switched to the first irradiation line **201**, the irradiation is performed from a first irradiation nozzle **210** provided to the first irradiation line. When delivery of the particle beam is switched to the second irradiation line **202**, the irradiation is performed from a second irradiation nozzle **220** provided to the second irradiation line.

Here, the first irradiation nozzle **210** is mounted with broad irradiation use parts, and the second irradiation nozzle **220** is mounted with scanning irradiation use parts. The broad irradiation is performed when the delivery line is switched to the first irradiation line, and the scanning irradiation is performed when the delivery line is switched to the second irradiation line.

When the irradiation line is switched to the first irradiation line or to the second irradiation line, no rotation of the gantry allows the broad irradiation and the scanning irradiation to be performed respectively from directions different by 180°.

In addition, when the irradiation line is switched, for example, from the first irradiation line to the second irradiation line, rotation of the gantry by 180 degrees allows the broad irradiation and the scanning irradiation to be performed from the same direction.

As described above, the broad irradiation and the scanning irradiation can be performed for one and the same patient, using an irradiation nozzle such as the irradiation nozzle **20**, shown in **FIGS. 2** and **3**, configured to be able to perform the broad irradiation and the scanning irradiation by switching parts provided to the irradiation nozzle, or the irradiation apparatus **200**, shown in **FIG. 4**, configured to be able to perform the broad irradiation and the scanning irradiation by switching between the first irradiation line **201** provided with the first irradiation nozzle **210** for the broad irradiation and the second irradiation line **202** provided with the second irradiation nozzle **220** for the broad irradiation.

While a dose monitor is provided in the irradiation nozzle to measure an irradiation dose of the particle beam during both scanning irradiation and broad irradiation, the way of controlling the dose is different between the scanning irradiation and the broad irradiation.

The scanning irradiation is performed by controlling particle beam irradiation doses imparted to respective irradiation points in a scanning irradiation region while shifting the particle beam, as with, for example, a spot scanning irradiation method in which irradiation is performed by repeating stop and shift of the particle beam.

While the broad irradiation is performed such as using the wobbler method in which the particle beam is spread by being scanned in a circular pattern or using a scattering method in which the particle beam is spread by being not shifted but scattered with a scatterer, either broad irradiation method does not control irradiation doses imparted to respective irradiation points in a broad irradiation region but controls the overall irradiation dose imparted to the entire broad irradiation region.

Next, a method of formulating a treatment plan is described, in which a target irradiation dose distribution is formed by imparting the sum of a broad irradiation dose and a scanning irradiation dose to the same irradiation object, i.e., a diseased site using the irradiation apparatus capable of switching between the scanning irradiation and the broad irradiation described above.

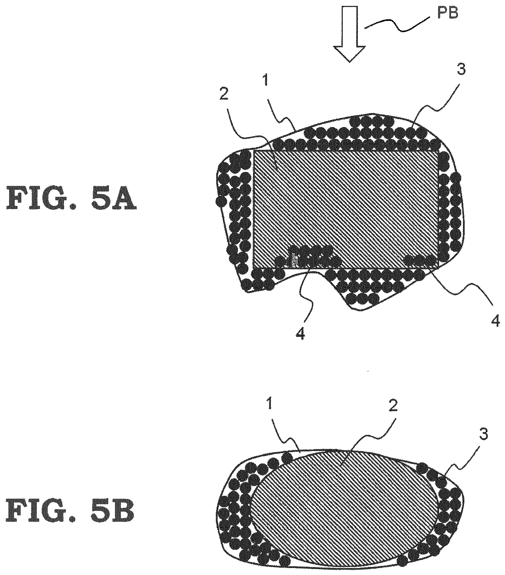

**FIGS. 5A** and **5B** show schematic illustrations of an irradiation based on a treatment plan formulated by the treatment planning apparatus according to Embodiment 1 of the present invention. **FIG. 5A** is a cross-sectional view of a diseased site, i.e., an irradiation region along the irradiation center axis of the particle beam *PB*; and **FIG. 5B** is a cross-sectional view orthogonal to the irradiation center axis.

First, a region to be subject to the broad irradiation is set as a broad irradiation region **2** in the diseased site, i.e., an irradiation region **1**. And then, a region, other than the broad irradiation region **2,** to be subject to the scanning irradiation is set as a scanning irradiation region **3** in the irradiation region **1**.

In Embodiment **1,** the broad irradiation is performed without using the bolus **44**. Performing the broad irradiation only using the ridge filter **42,** or the ridge filter **42** and the range shifter **43** without using the bolus **44** forms a broad irradiation region having a flat distal end and a flat proximal end as shown by, for example, the broad irradiation region **2** of **FIG. 5A.**

Controlling lateral range of the particle beam, for example, by the patient collimator **45** and by the scanning using the wobbler method allows the cross-section of the broad irradiation region **2** shown in **FIG. 5B** to be formed in various shapes. Thus, the broad irradiation region **2** is formed in a pillar shape.

For example, the broad irradiation region **2** is set so as to be inscribed in the irradiation region **1.** Briefly explaining, since this setting leaves in the irradiation region **1** an unirradiated region other than the broad irradiation region **2,** the region needs to be set as the scanning irradiation region **3** to be irradiated by the scanning irradiation. More strictly speaking, in the region to which an irradiation dose is imparted by the broad irradiation, there partially exist portions whose doses are unreached to their respective target irradiation doses.

In any case, a target irradiation dose distribution **Ds**(x, y, z) to be formed by the scanning irradiation can be calculated by subtracting a target irradiation dose distribution **Db** formed in the broad irradiation region by the broad irradiation from the target irradiation dose distribution **D** in the entire irradiation region, as expressed by the following Eq. (1):

$$D_s(x, y, z) = D(x, y, z) - D_b(x, y, z) \qquad (1)$$

As a result, the irradiation region **1** can be divided into

(1) a region irradiated only by the broad irradiation,
(2) regions irradiated only by the scanning irradiation, and
(3) regions (indicated by irradiation regions **4** shown in **FIG. 5A**) irradiated by both broad irradiation and scanning irradiation.

Formulation of a treatment plan based on a conventional scanning irradiation needs to solve an optimization problem to determine an irradiation angle, an irradiation dose for each irradiation point, further a scanning path (scanning trajectory) connecting each irradiation point, and the like to form the target irradiation dose distribution **D** in the entire irradiation region **1.**

A treatment plan formulated by the treatment planning apparatus according to the present invention only needs to solve an optimization problem for the target scanning irradiation dose distribution **Ds** calculated from the Eq. (1), thus reducing calculation time for the optimization. In addition, a conventional optimization technique (calculation algorism) can be used, as a matter of course, to form the target scanning irradiation dose distribution **Ds**.

As described before, the irradiation dose imparted by the broad irradiation is a so-called "fixed irradiation dose", and shortages of doses to be imparted by the scanning irradiation is "unfixed irradiation doses". Accordingly, the optimization is, in principle, to approximate the unfixed irradiation doses to the target irradiation doses as close as possible.

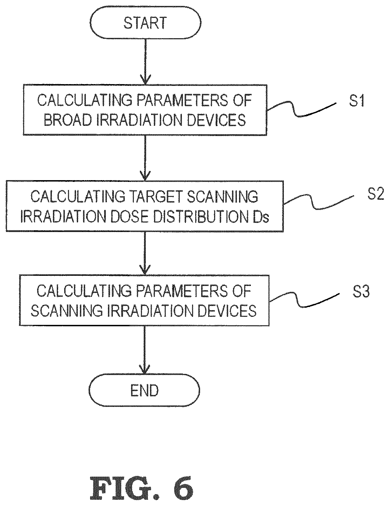

**FIG. 6** shows each of operation steps, i.e., an operation flow of the treatment planning apparatus. First, the broad irradiation parameter calculation unit **12** calculates the parameters for the respective devices relating to the broad irradiation in Step **S1** so that an irradiation dose imparted to every point by the broad irradiation does not exceed the target irradiation dose, in consideration that the broad irradiation region **2** is inscribed in the irradiation region **1** and the scanning irradiation further imparts irradiation doses to the points in the broad irradiation region **2.**

Next, the scanning irradiation parameter calculation unit **13** calculates the target scanning irradiation dose distribution **Ds** (x, y, z) according to Eq. (1) in Step **S2.** The scanning irradiation parameter calculation unit **13** further solves an optimization problem for the target irradiation dose distribution **Ds** to calculate parameters for the respective devices relating to the scanning irradiation in Step **S3.** These calculated parameters for the respective devices are sent to the overall device management apparatus **14.**

When starting treatment for a patient, i.e., starting the particle beam irradiation, the irradiation nozzle is, for example, first mounted with the parts for the broad irradiation, and then the respective devices are operated in accordance with their broad irradiation parameters sent from the overall device management apparatus **14**, so that the broad irradiation dose distribution **Db** is formed in the diseased site.

After that, the irradiation nozzle is mounted with the parts for the scanning irradiation, and then the respective devices are operated in accordance with their scanning irradiation parameters sent from the overall device management apparatus **14**, so that the scanning irradiation dose distribution **Ds** is formed in the diseased site.

By both irradiations, the irradiation dose distribution **D** = **Db** + **Ds** is imparted to the diseased site. Since the irradiation dose distribution only needs to satisfy **D** = **Db** + **Ds**, it is no matter which the broad irradiation or the scanning irradiation is performed first in order.

As described above, the treatment planning apparatus **10** formulates a treatment plan for both broad irradiation and scanning irradiation to form a target irradiation dose distribution in a diseased site, and a particle beam irradiation is performed in accordance with the treatment plan. This brings about the following effects.

The broad irradiation is a conventionally used irradiation method and has a merit in that there are many actual results in clinical practice; however, since broad irradiation regions are difficult to conform respectively to various shape diseased sites, a bolus is necessary for a broad irradiation region to conform to a diseased site shape on a patient-by-patient basis.

In contrast to that, the scanning irradiation is capable of forming various irradiation regions and various irradiation dose distributions by controlling the parameters for the respective devices; however, optimization calculation for the scanning irradiation takes time in formulating a treatment plan.

According to Embodiment **1** of the present invention, the broad irradiation imparts an irradiation dose to a large portion of an irradiation region and the scanning irradiation imparts irradiation doses to irradiation points in the remaining portions, which are mainly peripheral portions. This reduces the scanning irradiation regions thereby reducing time to formulate a treatment plan, and brings about an effect of being able to imparting irradiation doses to a diseased site having various shapes with high accuracy.

Furthermore, the scanning irradiation can be performed in a short time, thus facilitating performing the scanning irradiation, for example, with so-called respiration synchronizing control, i.e., during a less movement phase of a diseased site in the respiration cycles. Performing the scanning irradiation with the respiration synchronizing control brings about an effect of being able to impart irradiation doses with higher accuracy.

Embodiment **2**

In Embodiment **1**, no bolus is used in the broad irradiation. A bolus is usually fabricated for the range of the particle beam to conform to the shape of a diseased site, i.e., fabricated to adjust the energy distribution of the particle beam to the range conforming to a lower portion shape (distal shape) of a diseased site.

A broad irradiation using a bolus allows for forming an irradiation dose distribution in conformity to a distal shape of a diseased site. However, irradiation from one direction is difficult to form an irradiation dose distribution in conformity to both distal and proximal shapes of a diseased site, i.e., the shape of the entire diseased site.

Hence, a target irradiation dose distribution has been formed in the entire diseased site by a so-called multi-port irradiation such that irradiations are performed from, for example, an upper direction using a bolus for the distal shape and from the lower direction using a bolus for the proximal shape.

In Embodiment **2**, the broad irradiation is performed using a bolus, for example, a bolus for distal shape that forms an irradiation dose distribution in an irradiation region whose shape is the same as a distal shape of only a portion of an irradiation object, and the scanning irradiation is performed to impart irradiation doses to a portion to which the broad irradiation cannot impart an irradiation dose.

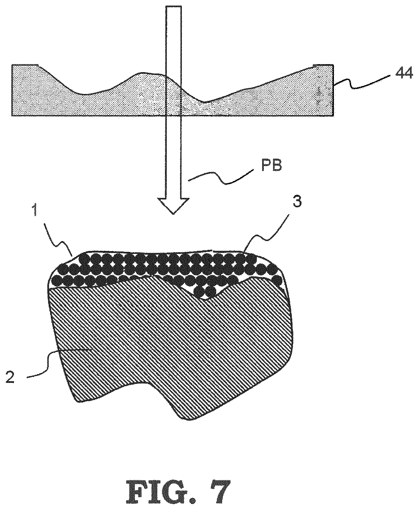

A schematic illustration of the irradiations is shown in **FIG. 7**. As shown in **FIG. 7,** a bolus **44** is used to form a broad irradiation region **2** conforming to the shape of a portion opposite to the incident side of the particle beam *PB*, i.e., conforming to the distal shape of an irradiation region **1.**

However, the broad irradiation using the bolus **44** alone cannot extend the broad irradiation region **2** to an irradiation region including a region conforming to the proximal shape of the diseased site. For that reason, the region in which the broad irradiation cannot form an irradiation dose distribution is regarded as a scanning irradiation region **3**, and the scanning irradiation imparts irradiation doses to the region.

In this case, it is sufficient to form an irradiation dose distribution only in the region proximal to the incident side of the particle beam by the scanning irradiation, thus allowing a treatment plan for the scanning irradiation to be formulated in a shorter time than Embodiment **1**.

Moreover, each irradiation of the scanning irradiation can be performed in a short time, thus facilitating performing the scanning irradiation, for example, in synchronism with respiration. Performing the scanning irradiation in synchronism with respiration brings about an effect of being able to impart irradiation doses with higher accuracy.

Embodiment **3**

In Embodiment **2**, the broad irradiation is performed using a bolus conforming to a distal shape of a portion of a diseased site. Conventionally, a bolus has been fabricated, each time on a patient-by-patient basis, as a patient-specific bolus conforming to the diseased site of a patient.

Embodiment **3** is characterized in that the broad irradiation is performed not using a patient-specific bolus but using a bolus that is selected to approximate to a distal shape of a diseased site among a plurality of device-specific different shape boluses prepared beforehand. The plurality of different shaped boluses is referred here to as versatile boluses.

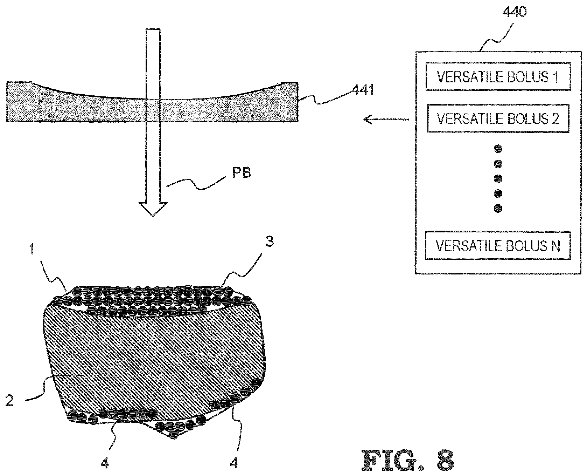

**FIG. 8** shows a schematic illustration of irradiation performed according to Embodiment **3** using a versatile bolus. For example, one versatile bolus **441** to be mounted to the irradiation nozzle for use in the broad irradiation is selected among N versatile boluses stored in a versatile bolus box **440.**

In a case of using one versatile bolus among the N versatile boluses beforehand prepared independently on the shape of a diseased site, the broad irradiation region is difficult to conform to the distal shape of the diseased site. A versatile bolus that forms the broad irradiation region **2** to be inscribed in the distal shape of a diseased site is preferably selected as the versatile bolus **441** among the plurality of versatile boluses. The broad irradiation is performed using the selected versatile bolus.

However, by the selected versatile bolus **441** alone, an irradiation dose cannot be imparted to the entire irradiation region **1.** For that reason, the regions in which the broad irradiation cannot form a target irradiation dose distribution are regarded as the scanning irradiation regions **3** and the irradiation region **4,** and the scanning irradiation imparts irradiation doses to the regions.

This reduces the scanning irradiation region **3** compared to Embodiment **1** that performs the broad irradiation without using a bolus, thus allowing a treatment plan for the scanning irradiation to be formulated in a further shorter time than Embodiment **1.**

Moreover, each irradiation of the scanning irradiation can be performed in a short time, thus facilitating performing the scanning irradiation, for example, in synchronism with respiration. Performing the scanning irradiation in synchronism with respiration brings about an effect of being able to impart an irradiation dose with higher accuracy.

List of Reference Signs

| | |
|---|---|
| **1** | irradiation region |
| **2** | broad irradiation region |
| **3** | scanning irradiation region |
| **10** | treatment planning apparatus |
| **11** | overall data management unit |
| **12** | broad irradiation parameter calculation unit |
| **13** | scanning irradiation parameter calculation unit |
| **20** | irradiation nozzle |
| **30** | accelerator |
| **44** | bolus |
| **210** | first irradiation nozzle |
| **220** | second irradiation nozzle |
| **441** | versatile bolus. |

**Claims**

**1.** A treatment planning apparatus configured to formulate a treatment plan that allows a particle beam therapy system to irradiate an irradiation object with a particle beam extracted from an accelerator, by switching between a scanning irradiation from a scanning irradiation nozzle mounted with scanning irradiation use parts for the scanning irradiation that is performed while shifting the particle beam and controlling an irradiation dose imparted to each of points in the irradiation object and a broad irradiation from a broad irradiation nozzle mounted with broad irradiation use parts for the broad irradiation that is performed by controlling a total irradiation dose imparted to a region in the irradiation object, the treatment planning apparatus comprising:

an overall data management unit configured to store a target irradiation dose distribution to be formed in the irradiation object;
a broad irradiation parameter calculation unit configured to calculate operational parameters for respective devices, such as the accelerator and the broad irradiation nozzle, to operate during the broad irradiation; and
a scanning irradiation parameter calculation unit configured to calculate operational parameters for the respective devices, such as the accelerator and the scanning irradiation nozzle, to operate during the scanning irradiation, wherein the broad irradiation parameter calculation unit and the scanning irradiation parameter calculation unit are adapted to cooperatively calculate and determine the operational parameters for the respective devices, such as the accelerator and the broad irradiation nozzle, to operate during the broad irradiation and the operational parameters for the respective devices, such as the accelerator and the scanning irradiation nozzle, to operate during the scanning irradiation, so that the sum of irradiation doses imparted by both broad irradiation and scanning irradiation forms the target irradiation dose distribution.

2. The treatment planning apparatus of claim **1**,
wherein the broad irradiation is performed without using a bolus.

3. The treatment planning apparatus of claim **1**,
wherein the broad irradiation is performed using a bolus for forming an irradiation dose distribution in an irradiation region whose shape is different from a distal shape of the entire irradiation object.

4. The treatment planning apparatus of claim **3**,
wherein the bolus forms an irradiation dose distribution in an irradiation region whose shape is the same as a distal shape of only a portion of the irradiation object.

5. The treatment planning apparatus of claim **3**,
wherein the broad irradiation use parts includes a plurality of device-specific boluses prepared beforehand, and the bolus is selected among the plurality of boluses.

6. A particle beam therapy system capable of irradiating an irradiation object with a particle beam extracted from an accelerator by switching between a scanning irradiation from a scanning irradiation nozzle mounted with scanning irradiation use parts for the scanning irradiation that is performed while controlling an irradiation dose imparted to each of points in the irradiation object and a broad irradiation from a broad irradiation nozzle mounted with broad irradiation use parts for the broad irradiation that is performed by controlling a total irradiation dose imparted to a region in the irradiation object, wherein respective devices, such as the accelerator, the scanning irradiation nozzle, and the broad irradiation nozzle, are controlled to operate in accordance with operational parameters determined for the respective devices by the treatment planning apparatus of any one of claims **1** through **5** .

FIG. 1

EP 3 281 673 A1

FIG. 2

12

EP 3 281 673 A1

FIG. 3

13

FIG. 4

FIG. 5A

FIG. 5B

START

CALCULATING PARAMETERS OF
BROAD IRRADIATION DEVICES — S1

CALCULATING TARGET SCANNING
IRRADIATION DOSE DISTRIBUTION Ds — S2

CALCULATING PARAMETERS OF
SCANNING IRRADIATION DEVICES — S3

END

# FIG. 6

FIG. 7

VERSATILE BOLUS 1

VERSATILE BOLUS 2

VERSATILE BOLUS N

FIG. 8

## INTERNATIONAL SEARCH REPORT

| | International application No. |
|---|---|
| | PCT/JP2015/061085 |

A. CLASSIFICATION OF SUBJECT MATTER
*A61N5/10*(2006.01)i

According to International Patent Classification (IPC) or to both national classification and IPC

B. FIELDS SEARCHED

Minimum documentation searched (classification system followed by classification symbols)
A61N5/10

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2010-29594 A  (National Institute of Radiological Sciences), 12 February 2010 (12.02.2010), entire text; all drawings (Family: none) | 1-6 |
| A | JP 2014-20800 A  (Sumitomo Heavy Industries, Ltd.), 03 February 2014 (03.02.2014), entire text; all drawings & US 2014/0014851 A1 | 1-6 |
| A | JP 2015-3259 A  (Mitsubishi Electric Corp.), 08 January 2015 (08.01.2015), entire text; all drawings (Family: none) | 1-6 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 June 2015 (15.06.15) | 30 June 2015 (30.06.15) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (July 2009)

**INTERNATIONAL SEARCH REPORT**

| International application No. |
| --- |
| PCT/JP2015/061085 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2013-17500 A  (Mitsubishi Electric Corp.),<br>31 January 2013 (31.01.2013),<br>entire text; all drawings<br>(Family: none) | 1-6 |
| A | JP 2006-128087 A  (Hitachi, Ltd.),<br>18 May 2006 (18.05.2006),<br>entire text; all drawings<br>& US 2006/0076515 A1    & EP 1642617 A1 | 1-6 |

Form PCT/ISA/210 (continuation of second sheet) (July 2009)

**REFERENCES CITED IN THE DESCRIPTION**

**Patent documents cited in the description**

- JP 2010158479 A
- JP 2009236867 A

- WO 2013011583 A1